Europäisches Patentamt

European Patent Office

Office européen des brevets

(11) Publication number: **0 057 607**

**B1**

(12) **EUROPEAN PATENT SPECIFICATION**

(45) Date of publication of patent specification: **29.08.84**

(21) Application number: **82300503.8**

(22) Date of filing: **01.02.82**

(51) Int. Cl.³: **C 07 C 5/27,** C 07 C 15/08, C 07 C 15/073, B 01 J 29/32

(54) **Xylene isomerization.**

(30) Priority: **02.02.81 US 230916**

(43) Date of publication of application:
**11.08.82 Bulletin 82/32**

(45) Publication of the grant of the patent:
**29.08.84 Bulletin 84/35**

(84) Designated Contracting States:
**BE DE FR GB IT NL**

(56) References cited:
**EP-A-0 020 043**
**EP-A-0 021 604**
**US-A-3 856 874**
**US-A-4 101 595**

(73) Proprietor: **MOBIL OIL CORPORATION**
**150 East 42nd Street**
**New York New York 10017 (US)**

(72) Inventor: **Chu, Yung Feng**
**121 Randle Drive**
**Cherry Hill New Jersey 08034 (US)**
Inventor: **Chester, Arthur Warren**
**517 County Club Drive**
**Cherry Hill New Jersey 08003 (US)**

(74) Representative: **West, Alan Harry et al**
**Mobil Court 3 Clements Inn**
**London WC2A 2EB (GB)**

EP 0 057 607 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for isomerizing the zylene content of a charge mixture of aromatic hydrocarbon compounds having eight carbon atoms with low xylene loss.

The present invention provides a process for isomerizing the xylene content of a charge mixture of aromatic hydrocarbon compounds having eight carbon atoms, which mixture contains xylenes and ethylbenzene by contact of the mixture and hydrogen at conversion conditions with a catalyst comprising a hydrogenation component and a crystalline zeolite having a silica to alumina ratio greater than 12 and a constraint index of 1 to 12, characterized by maintaining a conversion temperature of from 287° to 425°C (550° to 800°F), using a catalyst that has been steam treated to control its acid activity to a measured Alpha value of 45 to 110, and conducting the contacting at a space velocity effective to produce both a para xylene content of the xylenes at least 98% of that required for thermodynamic equilibrium and a predetermined percentage of conversion of the ethylbenzene content of the charge mixture.

Since the announcement of the first commercial installation of Octafining in Japan in June, 1958, this process has been widely installed for the supply of p-xylene. See "Advances in Petroleum Chemistry and Refining" volume 4 page 433 (Interscience Publishers, New York 1961). That demand for p-xylene has increased at remarkable rates, particularly because of the demand for terephthalic acid to be used in the manufacture of polyesters.

Typically, p-xylene is derived from mixtures of $C_8$ aromatics separated from such raw materials as petroleum naphthas, particularly reformates, usually by selective solvent extraction. The $C_8$ aromatics in such mixtures and their properties are:

|  | Freezing Point °F | Boiling Point °F |
| --- | --- | --- |
| Ethyl benzene | −139.0 | 277.1 |
| P-xylene | 55.9 | 281.0 |
| M-xylene | −54.2 | 282.4 |
| O-xylene | −13.3 | 292.0 |

Principal sources are catalytically reformed naphthas and pyrolysis distillates. The $C_8$ aromatic fractions from these sources vary quite widely in composition but will usually be in the range 2 to 32 wt.% ethylbenzene with the balance, xylenes, being divided approximately 50—65 wt.% meta, and the balance para and ortho.

Individual isomer products may be separated from the naturally occurring mixtures by appropriate physical methods. Ethylbenzene may be separated be fractional distillation although this is a costly operation. Ortho-xylene may be selected by fractional distillation and is so produced commercially. Para-xylene is separated from the mixed isomers by fractional crystallization or by selective adsorption.

As commercial use of para and ortho-xylene has increased there has been interest in isomerizing the other $C_8$ aromatics toward an equilibrium mix and thus increasing yields of the desired xylenes. At present, several xylene isomerization processes are available and in commercial use.

The isomerization process operates in conjunction with the product xylene or xylenes separation process. A virgin $C_8$ aromatics mixture is fed to such a processing combination in which the residual isomers emerging from the product separation steps are then charged to the isomerizer unit and the effluent isomerizate $C_8$ aromatics are recycled to the product separation steps. The composition of isomerizer feed is then a function of the virgin $C_8$ aromatic feed, the product separation unit performance, and the isomerizer performance.

It will be apparent that separation techniques for recovery of one or more xylene isomers will not have material effect on the ethylbenzene introduced with charge to the recovery/isomerization "loop". That compound, normally present in eight carbon atom aromatic fractions, will accumulate in the loop unless excluded from the charge or converted by some reaction in the loop to products which are separable from xylenes by means tolerable in the loop. Ethylbenzene can be separated from the xylenes of boiling point near that of ethylbenzene by extremely expensive "superfractionation". This capital and operating expense cannot be tolerated in the loop where the high recycle rate would require an extremely large distillation unit for the purpose. A charge preparation facility in which ethylbenzene is separated from the virgin $C_8$ aromatic fraction before introduction to the loop is a usual adjunct of low pressure, low temperature isomerization.

Other isomerization processes operate at higher pressure and temperature, usually under hydrogen pressure in the presence of catalysts which convert ethylbenzene to products readily separated by relatively simple distillation in the loop, which distillation is needed in any event to separate by-products of xylene isomerization from the recycle stream. For example, the Octafining catalyst of platinum on a silica-alumina composite exhibits the dual functions of hydrogenation/dehydrogenation and isomerization.

In Octafining, ethylbenzene reacts through ethyl cyclohexane to dimethyl cyclohexanes which in turn equilibrate to xylenes. Competing reactions are disproportionation of ethylbenzene to benzene and diethylbenzene, hydrocracking of ethylbenzene to ethylene and benzene and hydrocracking of the alkyl cyclohexanes.

The rate of ethylbenzene approach to equilibrium concentration in a $C_8$ aromatic mixture is related to effective contact time. Hydrogen partial pressure has a very significant effect on ethylbenzene approach to equilibrium. Temperature change within the range of Octafining conditions, i.e. (830°F to 900°F) has but a very small effect on ethylbenzene approach to equilibrium.

Concurrent loss of ethylbenzene to other molecular weight products relates to percent approach to equilibrium. Products formed from ethylbenzene include $C_6+$ naphthenes, benzene from cracking, benzene and $C_{10}$ aromatics from disproportion, and total loss to other than $C_8$ molecular weight. $C_5$ and lighter hydrocarbon by-products are also formed.

The three xylenes isomerize much more selectively than the reaction of ethylbenzene, but they do exhibit different rates of isomerization and hence, with different feed composition situations the rates of approach to equilibrium vary considerably.

Ethylbenzene has been found responsible for a relatively rapid decline in catalyst activity and this effect is proportional to its concentration in a $C_8$ aromatic feed mixture. It has been possible then to relate catalyst stability (or loss in activity) to feed composition (ethylbenzene content and hydrogen recycle ratio) so that for any $C_8$ aromatic feed, desired xylene products can be made with a selected suitably long catalyst use cycle.

A different approach to conversion of ethylbenzene is described in U.S. Patent No. 3,856,872. Over an active acid catalyst typified by zeolite ZSM-5, ethylbenzene disproportionates to benzene and diethylbenzene which are readily separated from xylenes by the distillation equipment needed in the loop to remove by-products. It is recognized that rate of disproportionation of ethylbenzene is related to the rate of conversion of xylenes to other compounds, e.g. by disproportionation. U.S. Patent No. 3,856,873 also describes the reaction of $C_8$ aromatics over ZSM-5 and shows effects of various temperatures up to 950°F in the absence of metal co-catalyst and in the absence of hydrogen.

In the known processes for accepting ethylbenzene to the loop, conversion of that compound is constrained by the need to hold conversion of xylenes to other compounds to an acceptable level. Thus, although the technique of U.S. Patent No. 3,856,872 provides significant advantages over Octafining in this respect, operating conditions are still selected to balance the advantages of ethylbenzene conversion against the disadvantages of xylene loss by disproportionation.

U.S. Patent No. 4,163,028 discloses zeolite isomerization and ethylbenzene conversion at high temperatures with ZSM-5 of very high silica to alumina ratio whereby the acid activity is reduced. U.S. Patent No. 4,236,996 discloses xylene isomerization concurrently with ethylbenzene conversion utilizing a certain steamed ZSM-5 catalyst. Conversion temperature of 700°F to 1000°F is described, the preferred conversion temperature being 800°F or higher. An improved method for preparation of platinum supported on ZSM-5 of controlled acidity is disclosed in U.S. application Serial No. 140,343 filed April 14, 1980 (Atty's Docket Number 0476).

The above recited art notwithstanding, there remain certain problems in the conversion of $C_8$ aromatics fractions which contain ethylbenzene. By their very nature, all of the proposed processes involve the high temperature isomerization of xylenes followed by reduction of temperature to crystallize para-xylene from the unwanted isomers, reheating of the recycle stream to high temperature and repetition of this cycle to exhaustively convert xylenes to para-xylene. Thus, the processes require considerable heat transfer with attendant heat losses and poor energy efficiency. It would be highly desirable to conduct the isomerization at temperatures below 800°F, such as in the temperature range of from 550°F to 800°F, to provide a process of improved energy efficiency. Additionally, the equilibrium content of para-xylene is higher at lower temperature, which favors overall efficiency of the process.

While the desirability of operation at lower temperatures has been recognized, $C_8$ fractions that contain ethylbenzene generally are not considered suitable for operation at less than 800°F due to excessive xylene loss by transalkylation with ethylbenzene at the lower temperatures. Operating at temperatures below 800°F also promotes xylene loss by disproportionation of xylenes if a high acid activity catalyst is used. Such loss may be minimized by operating with a low acidity platinum ZSM-5 catalyst, but this is not known to have been successfully used at low temperatures heretofore because, in general, such a catalyst did not have sufficient activity to convert the ethylbenzene. With inadequate activity for conversion of ethylbenzene, the residence time in the loop is increased, which tends to aggravate xylene loss by transalkylation.

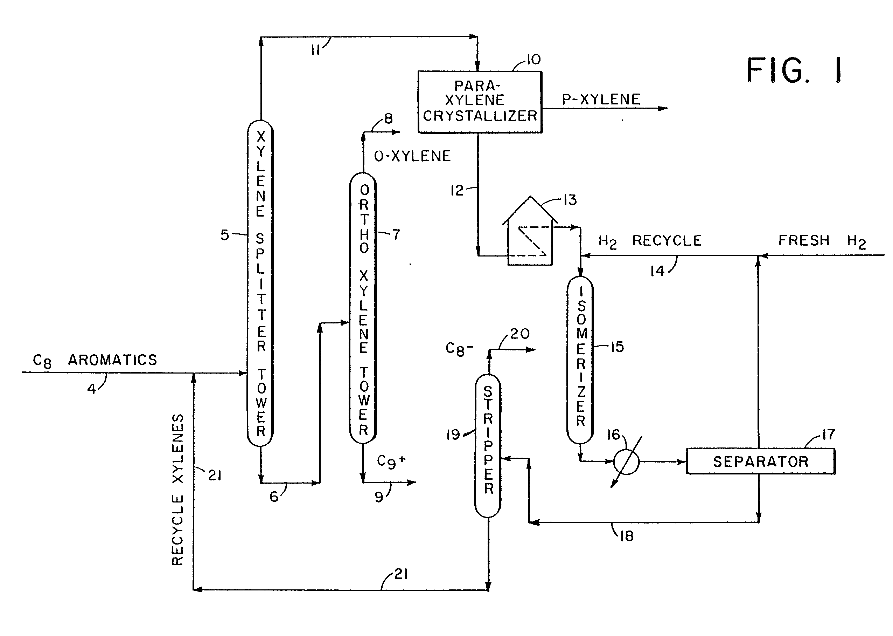

FIGURE 1. A plant suited to practice of the invention is illustrated as a diagrammatic flow-sheet.

This invention is based on the discovery that the xylene content or a mixture of aromatic hydrocarbons having eight carbon atoms that includes ethylbenzene is isomerized with conversion of ethylbenzene and with low loss of xylene at a temperature of from 550°F to 800°F, and preferably from 650°F to 750°F, by employing a zeolite catalyst exemplified by Pt-ZSM-5 that has been steam treated to control its acid activity to a preselected Alpha value in the range of from 45 to 110, as more fully described hereinbelow. Although steam treating is known to effect an advantageous deactivation of the ZSM-5 type catalyst for xylene isomerisation, as described in U.S. Patent No. 4,236,996, it is not believed heretofore known to steam-deactivate a zeolite catalyst to a controlled acidity and to employ it at a *lower* than usual temperature to effectively isomerize xylenes. The process provided by this invention readily forms a product mixture with a para-xylene content of the xylenes at least 98% of that required for thermodynamic equilibrium, and with an effectively reduced ethylbenzene content. Surprisingly, as will be seen from the examples, the ethylbenzene is converted primarily by hydrodealkylation to benzene, which is desirable.

It is particularly preferred for purposes of this invention to operate the process with a feed in which the paraffin content is low. Suitable fresh feed, for example, is the aromatic hydrocarbons fraction separated from an appropriate reformate cut by extraction.

The foregoing objects and advantages are obtained in a plant corresponding to the flow sheet in Figure 1. The charge introduced by line 4 is a mixture of alkyl aromatics having eight carbon atoms, namely ethylbenzene and the three xylene isomers. Such charge stocks are derived from catalytic reformates, pyrolysis gasoline, etc. by distillation and solvent extraction to separate aromatic compounds from aliphatics. Other sources for production of xylenes include toluene disproportionation and methylation of toluene. These charge stocks contain little or no ethylbenzene and therefore cannot take advantage of the novel ethylbenzene conversion feature of the invention. However, these are acceptable charge stocks alone or in combination with fractions which contain ethylbenzene. Such charge stock passes by line 4 to a xylene splitter column 5. The bottoms from the xylene splitter, constituted by o-xylene and $C_9$ aromatics passes by line 6 to the o-xylene tower 7 from which o-xylene is taken overhead at line 8 and heavy ends are removed by line 9. The overhead from xylene splitter column 5 is transferred to conventional crystallization separation 10 through line 11. The crystallizer may operate in the manner described in U.S. Patent No. 3,662,013.

Because its melting point is much higher than that of the other $C_8$ aromatics, p-xylene is readily separated in the crystallizer after refrigeration of the stream and a xylene mixture lean in p-xylene is transferred to an isomerization unit through line 12. The isomerization charge passes through a heater 13, is admixed with hydrogen admitted through line 14 and the mixture is introduced to the reactor 15 operated in a manner presently to be described.

Isomerized product from reactor 15 is cooled in heat exchanger 16 and passes to a high pressure separator 17 from which separated hydrogen can be recyled in the process. The liquid product of the isomerization passes by line 18 to a stripper 19 from which light ends are passed overhead by line 20. The remaining liquid product constituted by $C_8+$ hydrocarbons is recycled in the system by line 21 to the inlet of xylene stripper column 5.

It will be seen that the system is adapted to produce maximum quantities of p-xylene from a mixed $C_8$ aromatic feed containing all of the xylene isomers plus ethylbenzene. The key to efficient operation for that purpose is in the isomerizer which takes crystallizer effluent lean in p-xylene and converts the other xylene isomers in part to p-xylene for further recovery at the crystallizer.

The reactor 15 contains a crystalline alumino-silicate zeolite catalyst of controlled acid activity by reason of its treatment with steam at high temperatures. That catalyst, which is preferably combined with a metal from Group VIII of the Periodic Table, and most preferably with platinum, promotes xylene isomerization at a temperature of from 550°F to 800°F. Along with conversion of xylenes, ethylbenzene in the charge is selectively cracked to benzene and ethane at little or no conversion of xylenes to compounds other than xylenes, e.g., by disproportionation. This characteristic of the process renders unnecessary the preliminary distillation to separate at least some of the ethylbenzene from the feed stream as practiced in prior processes. However, it should be noted that the preferred raw feed to the process of this invention is what is commonly called an "extracted" feed, i.e., a feed substantially free of paraffins. The reason for this is that under the process condition herein described, the catalyst exhibits low activity for cracking branched chain hydrocarbons, which would build up in the loop and detract from efficient operation.

The flow sheet of FIGURE 1 contemplates separate recovery of o-xylene. It will be immediately apparent that this isomer may be recycled in the system in the event o-xylene is not a desired product. In that event, splitter tower 5 is operated to take o-xylene overhead with the other $C_8$ aromatics and take only $C_9+$ as bottoms from tower 5.

Particularly preferred are those zeolites having a constraint index within the approximate range of 1 to 12. Zeolites characterized by such constraint indices induce profound transformations of aliphatic hydrocarbons to aromatic hydrocarbons in commercially desirable yields and are generally highly effective in conversion reactions involving aromatic hydrocarbons. These zeolites retain their crystallinity for long periods in spite of the presence of steam at high temperature which induces irreversible collapse of the framework of other zeolites, e.g., of the X and A type. Furthermore, carbonaceous deposits, when formed, may be removed by burning at higher than usual temperatures to restore activity. In many environments the zeolites of this class exhibit very low coke forming capability, conducive to very long times on stream between burning regenerations.

An important characteristic of the crystal structure of this class of zeolites is that it provides constrained access to, and egress from the intra-crystalline free space by virtue of having a pore dimension greater than about 5 Angstroms and pore windows of about a size such as would be provided by 10-membered rings of oxygen atoms. It is to be understood, of course, that these rings are those formed by the regular disposition of the tetrahedra making up the anionic framework of the crystalline aluminosilicate, the oxygen atoms themselves being bonded to the silicon or aluminum atoms at the centers of the tetrahedra. Briefly, the preferred type zeolites useful in this invention possess, in combination, a silica to alumina mole ratio of at least about 12; and a structure providing constrained access to the crystalline free space.

The zeolite will have a silica/alumina ratio greater than 12. The silica to alumina ratio referred to may be determined by conventional analysis. This ratio is meant to represent, as closely as possible, the ratio in the rigid anionic framework of the zeolite crystal and to exclude aluminum in the binder or in cationic or other form within the channels. Such zeolites, after activation, acquire an intracrystalline sorption capacity for normal hexane which is greater than that for water, i.e., they exhibit "hydrophobic" properties. It is believed that this hydrophobic character is advantageous in the present invention.

The type of zeolites described freely sorb normal hexane and have a pore dimension greater than about 5 angstroms. In addition, the structure must provide constrained access to large molecules. It is sometimes possible to judge from a known crystal structure whether such constrained access exists. For example, if the only pore windows in a crystal are formed by 8-membered rings of oxygen atoms, then access by molecules of larger cross-section than normal hexane is excluded and the zeolite is not of the desired type. Windows of 10-membered rings are preferred, although, in some instances, excessive puckering or pore blockage may render these zeolites ineffective. Twelve-membered rings do not generally appear to offer sufficient constraint to produce the advantageous conversions, although puckered structures exist such as TMA offretite which is a known effective zeolite. Also, structures can be conceived, due to pore blockage or other cause, that may be operative.

Rather than attempt to judge from crystal structure whether or not a zeolite possesses the necessary constrained access, a simple determination of the "constraint index" may be made by passing continuously a mixture of an equal weight of normal hexane and 3-methylpentane over a sample of zeolite at atmospheric pressure according to the following procedure. A sample of the zeolite, in the form of pellets or extrudate, is crushed to a particle size about that of coarse sand and kept in a stream of air at 1000°F for at least 15 minutes. The zeolite is then flushed with helium and the temperature adjusted between 550°F and 950°F to give an overall hydrocarbon conversion between 10% and 60%. The mixture of hydrocarbons is passed at 1 liquid hourly space velocity (i.e., 1 volume of liquid hydrocarbon per volume of zeolite per hour) over the zeolite with a helium dilution to give a helium to (total) hydrocarbon mole ratio of 4:1. After 20 minutes on stream, a sample of the effluent is taken and analyzed, most conveniently by gas chromatography, to determine the fraction remaining unchanged for each of the two hydrocarbons.

The "Constraint Index" is calculated as follows:

$$\text{Constraint Index} = \frac{\log_{10}(\text{fraction of hexane remaining})}{\log_{10}(\text{fraction of 3-methylpentane remaining})}$$

The Constraint Index approximates the ratio of the cracking rate constants for the two hydrocarbons. Zeolites suitable for the present invention are those having a Constraint Index in the approximate range of 1 to 12.

**0 057 607**

| Zeolite | C.I. |
|---|---|
| ZSM-5 | 8.3 |
| ZSM-11 | 8.7 |
| ZSM-12 | 2 |
| ZSM-35 | 4.5 |
| ZSM-38 | 2 |
| TMA Offretite | 3.7 |
| Beta | 0.6 |
| ZSM-14 | 0.5 |
| H-Zeolon (mordenite) | 0.4 |
| REY | 0.4 |
| Amorphous Silica-Alumina | 0.6 |
| Erionite | 38 |

It is to be realized that the above constraint index values typically characterize the specified zeolites but that such are the cumulative result of several variables used in determination and calculation thereof. Thus, for a given zeolite depending on the temperatures employed within the aforenoted range of from 550°F to 950°F, with accompanying conversion between 10% and 60%, the constraint index may vary within the indicated approximate range of 1 to 12. Likewise, other variables such as the crystal size of the zeolite, the presence of possible occluded contaminants and binders intimately combined with the zeolite may affect the constraint index. It will accordingly by understood by those skilled in the art that the constraint index, as utilized herein, while affording a highly useful means for characterizing the zeolites of interest is approximate, taking into consideration the manner of its determination, with probability, in some instances, of compounding variables extremes.

While the above experimental procedure will enable one to achieve the desired overall conversion of 10 to 60% for most catalyst samples and represents preferred conditions, it may occasionally be necessary to use somewhat more severe conditions for samples of very low activity, such as those having a very high silica to alumina ratio or high sodium content. In those instances, a temperature of up to about 1000°F and a liquid hourly space velocity of less than one, such as 0.1 or less, can be employed in order to achieve a minimum total conversion of about 10%.

The class of zeolites defined herein is exemplified by ZSM-5, ZSM-11, ZSM-12, ZSM-35, ZSM-38, and other similar materials. ZMS-5 is described in U.S. Patent 3,702,886, ZSM-11 in U.S. Patent No. 3,709,979, ZSM-12 in U.S. Patent No. 3,832,449, ZSM-35 in U.S. Patent No. 4,016,245, and ZSM-38 in U.S. Patent No. 4,046,859.

The specific zeolites described, when prepared in the presence of organic cations, are catalytically inactive, possibly because the intra-crystalline free space is occupied by organic cations from the forming solution. They may be activated by heating in an inert atmosphere at 1000°F for one hour, for example, followed by base exchange with ammonium salts followed by calcination at 1000°F in air. The presence of organic cations in the forming solution may not be absolutely essential to the formation of this type zeolite; however, the presence of these cations does appear to favor the formation of this special type of zeolite. More generally, it is possible (and is usual practice) to activate this type catalyst by base exchange with ammonium salts followed by calcination in air at about 1000°F for from about 15 minutes to about 24 hours.

Natural zeolites may sometimes be converted to this type zeolite catalyst by various activation procedures and other treatments such as base exchange, steaming, alumina extraction and calcination, in combinations. Natural minerals which may be so treated include ferrierite, brewsterite, stilbite, dachiardite, epistilbite, heulandite, and clinoptilolite.

The preferred crystalline alumino-silicate are ZSM-5, ZSM-11, ZSM-12, ZSM-35, and ZSM-38 with ZSM-5 particularly preferred.

In a preferred aspect of this invention, the zeolites hereof are selected as those having a crystal framework density, in the dry hydrogen form, of not less than about 1.6 grams per cubic centimeter. It has been found that zeolites which satisfy all three of these criteria are most desired. Therefore, the preferred zeolites of this invention are those having a Constraint Index as defined above of about 1 to

6

about 12, a silica to alumina mole ratio of at least about 12 and a dried crystal density of not less than about 1.6 grams per cubic centimeter. The dry density for known structures may be calculated from the number of silicon plus aluminum atoms per 1000 cubic Angstroms, as given in the article ZEOLITE STRUCTURE by W. M. Meier, PROCEEDINGS OF THE CONFERENCE ON MOLECULAR SIEVES, (London, April 1967) published by the Society of Chemical Industry, London, 1968, page 19.

When the crystal structure is unknown, the crystal framework density may be determined by classical pycnometer techniques. For example, it may be determined by immersing the dry hydrogen form of the zeolite in an organic solvent which is not sorbed by the crystal. It is possible that the unusual sustained activity and stability of this class of zeolites is associated with its high crystal anionic framework density of not less than about 1.6 grams per cubic centimeter. This high density, of course, must be associated with a relatively small amount of free space within the crystal, which might be expected to result in more stable structures. This free space, however, is important as the locus of catalytic activity.

Crystal framework densities of some typical zeolites are:

|  | Void Volume | Framework Density |
|---|---|---|
|  | cc/cc | g/cc |
| Ferrierite | 0.28 | 1.76 |
| Mordenite | 0.28 | 1.7 |
| ZSM-5, -11 | 0.29 | 1.79 |
| Dachiardite | 0.32 | 1.72 |
| L | 0.32 | 1.61 |
| Clinoptilolite | 0.34 | 1.71 |
| Laumontite | 0.34 | 1.77 |
| ZSM-4 (Omega) | 0.38 | 1.65 |
| Heulandite | 0.39 | 1.69 |
| P | 0.41 | 1.57 |
| Offretite | 0.40 | 1.55 |
| Levynite | 0.40 | 1.54 |
| Erionite | 0.35 | 1.51 |
| Gmelinite | 0.44 | 1.46 |
| Chabazite | 0.47 | 1.45 |
| A | 0.5 | 1.3 |
| Y | 0.48 | 1.27 |

When synthesized in the alkali metal form, the zeolite may be converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been replaced by another cation may be used. Thus, the original alkali metal of the zeolite may be replaced by ion exchange with other suitable ions of Groups IB and VIII of the Periodic Table, including, by way of example, nickel, copper, zinc, palladium, calcium or rare earth metals.

In practicing the desired conversion process, it may be desirable to incorporate the above-described crystalline aluminosilicate zeolite in another material resistant to the temperature and other conditions employed in the process. Such matrix materials include synthetic or naturally occurring sub-

7

stances as well as inorganic materials such as clays, silica and/or metal oxides. The latter may be either naturally occurring in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-berylia, silica-titania as well as ternary compositions, such as silica-alumina-thoria, silica-alumina-zirconia silica-alumina-magnesia and silica-magnesia-zirconia. The matrix may be in the form of a cogel. The relative proportions of zeolite component and inorganic oxide gel matrix may vary widely with the zeolite content ranging from between about 1 to about 99 percent by weight and more usually in the neighborhood of about 65 percent by weight of the composite.

For purposes of the present invention, a hydrogenation component is present with the zeolite catalyst. This component may be added to the catalyst by ion exchange, impregnation, or any other means known in the art and at any stage of catalyst preparation. The hydrogenation component may comprise a Group VIII metal, alone or in association with a Group VI A metal, the metal or metals being in highly dispersed elemental form or as oxides, sulfides or as other compounds. References herein to groups of the periodic table are to be understood to refer to the "Periodic Chart of the Elements", published by Fisher Scientific Company, Copyright 1978, Cat. No. 5-702-10. In a particularly preferred embodiment of the process of this invention, the hydrogenation component consists essentially of platinum which is present in an amount of from 0.05 to 1.5 weight percent of the catalyst, the platinum having been incorporated with the catalyst by the method described in U.S. Application Serial No. 140,343 filed April 14, 1980 (Atty's Docket 00 0476). In essence, the method comprises incorporating the metal in cationic form with the crystalline zeolite prior to final catalyst particle formation.

It is considered essential for purposes of this invention to steam the catalyst prior to use in the process of this invention to control its acid activity. In particular, the unsteamed catalyst, which may be referred to herein as the precursor catalyst, is steamed under relatively wild conditions, such as for about one to about ten hours with 100% steam at atmospheric pressure and at a temperature of from 800°F to 1000°F to reduce its acidity to a measured "Alpha" value in the range of from 45 to 110. It is to be understood, of course, that the treating conditions may be altered from those recited such as by using superatmospheric pressure at reduced temperature, the critical parameter being the controlled acidity of the steamed catalyst. In any case, however, sufficient steam treatment is required to reduce Alpha by at least 10.

The controlled acid activity of the catalyst is conveniently defined by the "Alpha" scale described in an article published in Journal of Catalysis, Vol. VI, pp. 278—287 (1966). In this test, the catalyst is contacted with hexane under prescribed conditions and the amount of hexane which is cracked is measured. From this measurement is computed the "Alpha" value used herein. For purposes of the present invention, however, all measurements of "Alpha" value are to be made at 1000°F, and all references to "Alpha" value are to be understood to refer to the value obtained when the hexane cracking is measured at 1000°F.

In general, it is desirable to operate the process of this invention in the presence of hydrogen gas to extend catalyst life and maintain good efficiency. The hydrogen to hydrocarbon mole ratio may be from 0.1 to 10, and preferably from 0.5 to 5.0, with a total pressure from about atmospheric up to 1000 psig. The space velocity is usually adjusted to provide a desired conversion of ethylbenzene per cycle, usually from about 20 to 40%, and with at least 98% para approach to equilibrium.

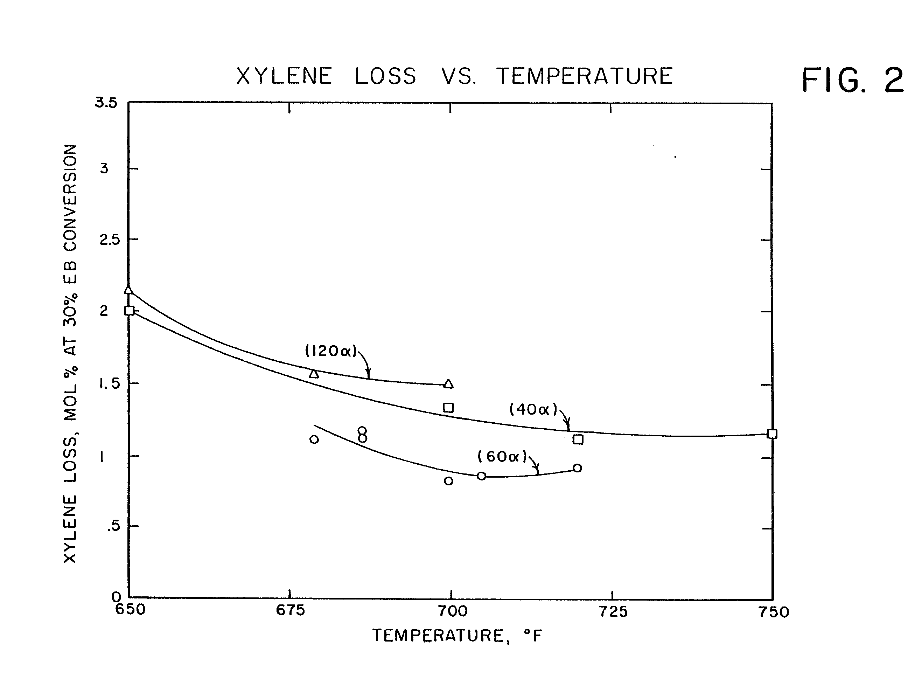

Figure 2 compares xylene loss vs. temperature. The data shown are from Examples 9 through 27 below excluding Example 11. This Figure shows that by conjunctively correlating catalyst activity and process conditions, the process of this invention may be conducted with very low loss of xylenes and at a temperature significantly below 800°F, thus providing unusually good energy efficiency. From Figure 2, it is evident that, given a particular feed and a predetermined percentage conversion of ethylbenzene per pass, that not only is there an optimal Alpha value in the range of from 45 to 110 for which particularly low xylene loss is obtainable, but that there is also an optimum temperature within the range of from 650°F to 750°F that gives particularly low loss of xylenes for the optimal Alpha value. It is contemplated that the particular values of Alpha value and temperature which minimize xylene losses are subject to some variation depending on the feed composition and the prescribed level of ethylbenzene conversion, and possibly also depending on process variables such as hydrogen to hydrocarbon mole ratio and total pressure. Therefore, it is contemplated as within the ambit of this invention and as an embodiment thereof to select at least the desired level of ethylbenzene conversion and a prescribed feed composition, approximating the recycle plus fresh feed in the loop entering the isomerizer, and to estimate from a series of measurements, the optimal Alpha value of the catalyst in the range of from 45 to 110, and the optimal temperature in the range of from 650°F to 750°F for a catalyst of optimal Alpha value and to operate the process with that catalyst at about the optimal temperature. This embodiment of the present invention provides minimum xylene loss, but not necessarily the

0057607

minimum operating temperature.

The following examples illustrate the present invention. Examples 1 to 4 illustrate the preparation of catalyst both within and outside of the scope of this invention. All parts are by weight unless otherwise stated.

## Example 1

HZSM-5 crystalline zeolite powder having a 70:1 silica/alumina ratio is contacted with a solution of tetrammine platinum chloride in an amount calculated to deposit 0.1 wt.% of platinum on the final catalyst. An amount of Alpha-alumina mono-hydrate is added to the zeolite to provide a final catalyst having about 50 wt.% content of zeolite on an anhydrous basis. The zeolite and matrix are intimately mixed and tempered with water and extruded to form pellets. The pellets are dried and then they are calcined in nitrogen to remove the organics. Then they are exchanged with aqueous ammonium nitrate to reduce the sodium content to below 0.05 wt.%. The exchanged pellets are dried and then calcined again in air.

A portion of the calcined pellets are exposed to 100% steam at atmospheric pressure and at a temperature of 1025°F for 24 hours. The Alpha value of the steamed catalyst, measured at 1000°F, was 10. This catalyst is not within the scope of this invention.

## Example 2

Calcined pellets prepared as in Example 1 were steamed as in Example 1 but for 2.5 hours at 1000°F. The measured Alpha value was 40. This catalyst is not within the scope of this invention.

## Example 3

Calcined pellets prepared as in Example 1 were steamed as in Example 1 but for 4.5 hours at 900°F. The measured Alpha value was 60. This catalyst is within the scope of this invention.

## Example 4

Calcined pellets prepared as in Example 1 were steamed as in Example 1 but for 4 hours at 850°F. The measured Alpha value was 120. This catalyst is not within the scope of this invention.

Examples 5 to 28 illustrate the isomerization of a mixed xylene feed that contains ethylbenzene, utilizing the catalysts prepared in Examples 1 to 4. The results are summarized in Tables 1 to 5, inclusive. The xylene losses shown in the tables are computed by adding the appearance (or disappearance) of toluene, trimethylbenzene and ethyl xylene. Thus,

$$\text{Xylene Loss, Mol Pct} = \frac{[T]_{P-F} + [TMB]_{T-F} + [EX]_{P-F}}{[pX + mX + oX]_F} \times 100$$

wherein [ ] = Mol Pct

T = Toluene

TMB = Trimethyl benzenes

EX = Ethyl Xylenes

P = product

F = feed

pX, mX, and oX = para, meta, and ortho xylene, resp.

The equilibrium approach values for the xylenes as shown in the table are a measure of the extent of conversion compared with conversion expected with all three isomers in thermodynamic equilibrium. The xylenes composition is normalized for this calculation. Values greater than 100% are possible with incomplete equilibration. A typical computation is made as follows:

$$\text{Equilibrium Approach, pX, wt.\%,} = \frac{(pX)_P - (pX)_F}{(pX)_E - (pX)_F} \times 100$$

wherein ( ) represents wt.% of normalized xylenes composition, E represents equilibrium value for a given temperature of reaction, and the other symbols are as represented above.

9

# 0 057 607

TABLE 1
Xylene Isomerization

| Example No. | | 5 | 6 | 7 | 8 |
|---|---|---|---|---|---|
| Catalyst | | ← ——— Example 1 ———→ | | | |
| Temperature, °F | | 853 | 853 | 854 | 851 |
| Pressure, PSIG | | 100 | 100 | 100 | 100 |
| WHSV | | 10.1 | 14.7 | 5.0 | 19.8 |
| $H_2$/HC Molar Ratio | | 2.3 | 2.2 | 2.2 | 2.3 |
| Time on Stream, Hrs. | | 22.0 | 46.0 | 23.5 | 20.0 |
| EB Conversion, Wt. Pct. | | 46.3 | 35.3 | 69.9 | 25.4 |
| Xylene Loss, Mol. Pct. | | 2.0 | 1.4 | 3.5 | 0.9 |
| Ring Loss, Mol. Pct. | | 0.0 | −0.1 | 0.0 | 0.0 |
| Equilibrium Approach | | | | | |
| P-Xylene | | 107.0 | 104.9 | 106.1 | 98.5 |
| M-Xylene | | 99.4 | 94.6 | 94.7 | 88.1 |
| O-Xylene | | 63.9 | 46.8 | 41.8 | 39.2 |
| $C_2$ = /$C_2$ Molar Ratio | | 0.01 | 0.02 | 0.01 | 0.02 |
| $C_2$/$\Delta$EB Molar Ratio | | 0.73 | 0.77 | 0.74 | 0.74 |
| $\Delta$BZ/$\Delta$EB Molar Ratio | | 1.10 | 1.06 | 0.91 | 1.02 |
| $H_2$ Balance | | 96.8 | 99.7 | 98.0 | 99.9 |
| Carbon Balance | | 95.8 | 99.8 | 97.1 | 99.7 |
| Ring Balance | | 95.9 | 99.9 | 97.2 | 99.8 |
| $C_2$ Balance | | 89.9 | 95.3 | 85.5 | 97.3 |
| Total Balance | | 95.9 | 99.8 | 97.2 | 99.7 |
| Product Distribution, Wt. Pct. | Feed | | | | |
| $C_1$—$C_4$ N.A. | | 0.9 | 0.7 | 1.3 | 0.5 |
| $C_5$—$C_9$ N.A. | | 0.1 | 0.0 | 0.0 | 0.0 |
| Benzene | | 3.0 | 2.2 | 3.8 | 1.5 |
| Toluene | 1.5 | 2.5 | 2.1 | 3.0 | 1.8 |
| EB | 8.1 | 4.3 | 5.2 | 2.4 | 6.0 |

10

**0 057 607**

TABLE 1—continued

| Example No. | 5 | 6 | 7 | 8 |
|---|---|---|---|---|
| P-Xylene | 9.2 | 21.4 | 21.3 | 21.2 | 20.7 |
| M-Xylene | 61.3 | 45.7 | 46.8 | 46.2 | 48.0 |
| O-Xylene | 19.9 | 21.0 | 20.7 | 20.3 | 20.7 |
| $C_9$+ Arom. | | 1.1 | 0.9 | 1.8 | 0.7 |
| TOTAL | 100.0 | 100.0 | 100.0 | 100.0 | 100.0 |

TABLE 2
Xylene Isomerization

| Example No. | 9 | 10 | 11 | 12 | 13 | 14 |
|---|---|---|---|---|---|---|
| Catalyst | ← | | Example 2 | | | → |
| Temperature, °F | 700 | 700 | 650 | 650 | 720 | 750 |
| Pressure, PSIG | 100 | 100 | 100 | 100 | 100 | 100 |
| WHSV | 7.0 | 7.0 | 7.0 | 1.9 | 9.9 | 13.9 |
| $H_2$/HC Molar Ratio | 2.0 | 2.0 | 2.0 | 2.0 | 2.0 | 2.0 |
| Time on Stream, Hrs. | 4.7 | 23.8 | 28.8 | 35.3 | 39.8 | 46.3 |
| EB Conversion, Wt. Pct. | 34.7 | 31.0 | 11.4 | 36.7 | 33.9 | 35.7 |
| Xylene Loss, Mol. Pct. | 1.5 | 1.4 | 0.7 | 2.0 | 1.1 | 1.2 |
| Ring Loss, Mol. Pct. | 0.5 | 0.0 | 0.0 | −0.4 | 0.0 | −0.1 |
| Equilibrium Approach | | | | | | |
| P-Xylene | 103.5 | 104.5 | 102.8 | 103.4 | 102.4 | 104.5 |
| M-Xylene | 94.5 | 96.2 | 94.8 | 95.5 | 93.7 | 94.8 |
| O-Xylene | 15.3 | 24.0 | −11.1 | −9.1 | 25.8 | 29.7 |
| $C_2$=/$C_2$ Molar Ratio | 0.00 | 0.00 | 0.00 | 0.00 | 0.01 | 0.01 |
| $C_2$/$\Delta$EB Molar Ratio | 0.71 | 0.61 | 0.22 | 0.07 | 0.59 | 0.65 |
| $\Delta$BZ/$\Delta$EB Molar Ratio | 0.92 | 0.97 | 1.08 | 0.92 | 0.92 | 0.97 |
| $H_2$ Balance | 100.0 | 98.8 | 100.0 | 96.1 | 98.9 | 98.9 |
| Carbon Balance | 99.3 | 99.3 | 99.2 | 96.3 | 98.7 | 99.1 |
| Ring Balance | 98.9 | 99.2 | 99.3 | 96.7 | 98.7 | 99.1 |
| $C_2$ Balance | 97.7 | 95.2 | 97.6 | 77.2 | 94.4 | 95.0 |
| Total Balance | 99.4 | 99.2 | 99.3 | 96.3 | 98.7 | 99.0 |

11

**0 057 607**

TABLE 2—continued

| Example No. | | 9 | 10 | 11 | 12 | 13 | 14 |
|---|---|---|---|---|---|---|---|
| Product Distribution, Wt. Pct. | Feed | | | | | | |
| $C_1$—$C_4$ N.A. | | 1.0 | 0.6 | 0.1 | 0.1 | 0.6 | 0.6 |
| $C_5$—$C_9$ N.A. | | 0.1 | 0.0 | 0.0 | 0.0 | 0.0 | 0.0 |
| Benzene | | 1.9 | 1.8 | 0.7 | 2.0 | 1.9 | 2.1 |
| Toluene | 1.5 | 2.0 | 1.9 | 1.7 | 2.1 | 1.8 | 1.9 |
| EB | 8.1 | 5.3 | 5.6 | 7.2 | 5.1 | 5.3 | 5.2 |
| P-Xylene | 9.2 | 21.3 | 21.6 | 21.6 | 21.5 | 21.3 | 21.5 |
| M-Xylene | 61.3 | 47.4 | 47.5 | 48.3 | 47.8 | 47.9 | 47.5 |
| O-Xylene | 19.9 | 19.7 | 19.9 | 19.6 | 19.5 | 20.1 | 20.2 |
| $C_9$+ Arom. | | 1.3 | 1.2 | 0.8 | 1.9 | 1.1 | 1.1 |
| TOTAL | 100.0 | 100.0 | 100.0 | 100.0 | 100.0 | 100.0 | 100.0 |

TABLE 3
Xylene Isomerization

| Example No. | 15 | 16 | 17 | 18 | 19 |
|---|---|---|---|---|---|
| Catalyst | ← | | Example 3 | | → |
| Temperature, °F | 750 | 700 | 720 | 680 | 687 |
| Pressure, PSIG | 100 | 100 | 100 | 100 | 100 |
| WHSV | 6.9 | 7.0 | 14.0 | 7.0 | 7.0 |
| $H_2$/HC Molar Ratio | 2.0 | 2.0 | 2.0 | 2.0 | 2.0 |
| Time on Stream, Hrs. | 20.5 | 43.5 | 63.0 | 84.5 | 89.9 |
| EB Conversion, Wt. Pct. | 70.8 | 40.6 | 30.9 | 28.6 | 33.2 |
| Xylene Loss, Mol. Pct. | 3.0 | 1.5 | 1.0 | 1.1 | 1.2 |
| Ring Loss, Mol. Pct. | 0.2 | 0.1 | −0.1 | 0.0 | 0.1 |
| Equilibrium Approach | | | | | |
| P-Xylene | 100.8 | 102.1 | 101.8 | 102.6 | 101.2 |
| M-Xylene | 91.7 | 93.0 | 92.2 | 94.4 | 94.5 |
| O-Xylene | 30.3 | 12.9 | 17.8 | 10.7 | 30.8 |

12

TABLE 3—continued

| Example No. | 15 | 16 | 17 | 18 | 19 |
|---|---|---|---|---|---|
| $C_2=/C_2$ Molar Ratio | 0.00 | 0.00 | 0.00 | 0.00 | 0.00 |
| $C_2/\Delta EB$ Molar Ratio | 0.82 | 0.73 | 0.65 | 0.53 | 0.56 |
| $\Delta BZ/\Delta EB$ Molar Ratio | 0.94 | 0.97 | 0.94 | 0.94 | 0.91 |
| $H_2$ Balance | 100.1 | 100.2 | 99.8 | 99.6 | 99.7 |
| Carbon Balance | 99.9 | 100.0 | 100.1 | 99.4 | 99.3 |
| Ring Balance | 99.8 | 100.0 | 100.1 | 99.4 | 99.3 |
| $C_2$ Balance | 91.3 | 96.2 | 95.3 | 94.6 | 92.9 |
| Total Balance | 99.9 | 100.0 | 100.1 | 99.4 | 99.3 |

| Product Distribution, Wt. Pct. | Feed | | | | | |
|---|---|---|---|---|---|---|
| $C_1$—$C_4$ N.A. | | 1.6 | 0.8 | 0.5 | 0.4 | 0.5 |
| $C_5$—$C_9$ N.A. | | 0.1 | 0.0 | 0.0 | 0.0 | 0.0 |
| Benzene | | 3.9 | 2.3 | 1.7 | 1.6 | 1.8 |
| Toluene | 1.5 | 2.8 | 2.1 | 1.8 | 1.8 | 1.9 |
| EB | 8.1 | 2.4 | 4.8 | 5.6 | 5.8 | 5.4 |
| P-Xylene | 9.2 | 20.7 | 21.2 | 21.3 | 21.4 | 21.3 |
| M-Xylene | 61.3 | 47.1 | 47.8 | 48.2 | 48.0 | 48.0 |
| O-Xylene | 19.9 | 19.8 | 19.7 | 20.0 | 19.8 | 20.0 |
| $C_9+$ Arom. | | 1.6 | 1.1 | 0.9 | 1.1 | 1.1 |
| TOTAL | 100.0 | 100.0 | 100.0 | 100.0 | 100.0 | 100.0 |

TABLE 4
Xylene Isomerization

| Example No. | 20 | 21 | 22 | 23 | 24 |
|---|---|---|---|---|---|
| Catalyst | ← | | Example 3 | | → |
| Temperature, °F | 687 | 700 | 700 | 705 | 705 |
| Pressure, PSIG | 100 | 100 | 100 | 100 | 100 |
| WHSV | 6.9 | 10.0 | 10.0 | 10.0 | 10.0 |
| $H_2$/HC Molar Ratio | 2.0 | 2.0 | 2.0 | 2.0 | 2.0 |
| Time on Stream, Hrs. | 157.5 | 178.5 | 202.5 | 226.5 | 250.8 |

13

**0 057 607**

TABLE 4—continued

| Example No. | | 20 | 21 | 22 | 23 | 24 |
|---|---|---|---|---|---|---|
| EB Conversion, Wt. Pct. | | 32.9 | 27.9 | 27.3 | 28.4 | 27.9 |
| Xylene Loss, Mol. Pct. | | 1.1 | 0.9 | 0.9 | 0.9 | 0.9 |
| Ring Loss, Mol. Pct. | | −0.1 | 0.1 | 0.0 | −0.1 | 0.0 |
| Equilibrium Approach | | | | | | |
| P-Xylene | | 100.0 | 101.8 | 101.2 | 101.9 | 102.2 |
| M-Xylene | | 95.9 | 92.9 | 92.1 | 92.5 | 92.8 |
| O-Xylene | | 56.4 | 15.1 | 11.5 | 12.4 | 13.2 |
| $C_2=/C_2$ Molar Ratio | | 0.00 | 0.00 | 0.00 | 0.00 | 0.00 |
| $C_2/\Delta EB$ Molar Ratio | | 0.50 | 0.72 | 0.64 | 0.48 | 0.66 |
| $\Delta BZ/\Delta EB$ Molar Ratio | | 0.91 | 0.96 | 0.98 | 0.96 | 1.00 |
| $H_2$ Balance | | 99.0 | 100.2 | 100.5 | 100.1 | 99.8 |
| Carbon Balance | | 99.0 | 99.9 | 100.2 | 99.6 | 99.8 |
| Ring Balance | | 99.0 | 99.8 | 100.2 | 99.7 | 99.8 |
| $C_2$ Balance | | 90.8 | 98.9 | 96.8 | 91.9 | 97.2 |
| Total Balance | | 99.0 | 99.9 | 100.2 | 99.7 | 99.8 |
| Product Distribution, Wt. Pct. | Feed | | | | | |
| $C_1$—$C_4$ N.A. | | 0.5 | 0.6 | 0.5 | 0.4 | 0.5 |
| $C_5$—$C_9$ N.A. | | 0.0 | 0.0 | 0.0 | 0.0 | 0.0 |
| Benzene | | 1.8 | 1.6 | 1.6 | 1.6 | 1.7 |
| Toluene | 1.5 | 1.8 | 1.8 | 1.8 | 1.8 | 1.8 |
| EB | 8.1 | 5.4 | 5.8 | 5.9 | 5.8 | 5.8 |
| P-Xylene | 9.2 | 21.2 | 21.3 | 21.3 | 21.4 | 21.4 |
| M-Xylene | 61.3 | 47.8 | 48.1 | 48.3 | 48.3 | 48.1 |
| O-Xylene | 19.9 | 20.4 | 19.9 | 19.8 | 19.9 | 19.9 |
| $C_9$+ Arom. | | 1.1 | 0.9 | 0.9 | 0.9 | 0.9 |
| TOTAL | 100.0 | 100.0 | 100.0 | 100.0 | 100.0 | 100.0 |

14

**0 057 607**

TABLE 5
Xylene Isomerization

| Example No. | | 25 | 26 | 27 | 28 |
|---|---|---|---|---|---|
| Catalyst | | ← | | Example 4 | → |
| Temperature, °F | | 650 | 680 | 700 | 720 |
| Pressure, PSIG | | 100 | 100 | 100 | 100 |
| WHSV | | 7.0 | 10.0 | 13.9 | 13.9 |
| $H_2$/HC Molar Ratio | | 2.0 | 2.0 | 2.0 | 2.0 |
| Time on Stream, Hrs. | | 20.5 | 42.5 | 63.0 | 67.0 |
| EB Conversion, Wt. Pct. | | 29.8 | 35.6 | 34.5 | 43.8 |
| Xylene Loss, Mol. Pct. | | 2.2 | 1.6 | 1.5 | 1.7 |
| Ring Loss, Mol. Pct. | | 0.4 | 0.2 | −0.2 | −0.1 |
| Equilibrium Approach | | | | | |
| P-Xylene | | 104.0 | 101.4 | 104.6 | 102.1 |
| M-Xylene | | 96.0 | 96.9 | 95.6 | 99.0 |
| O-Xylene | | −9.0 | 51.3 | 16.8 | 74.7 |
| $C_2 = /C_2$ Molar Ratio | | 0.00 | 0.01 | 0.01 | 0.01 |
| $C_2/\Delta EB$ Molar Ratio | | 0.63 | 0.51 | 0.60 | 0.71 |
| $\Delta BZ/\Delta EB$ Molar Ratio | | 0.86 | 0.85 | 0.94 | 0.89 |
| $H_2$ Balance | | 99.5 | 100.6 | 97.4 | 96.8 |
| Carbon Balance | | 99.3 | 99.0 | 99.0 | 98.8 |
| Ring Balance | | 98.9 | 99.0 | 99.0 | 98.6 |
| $C_2$ Balance | | 101.5 | 95.8 | 97.1 | 97.4 |
| Total Balance | | 99.3 | 99.2 | 98.8 | 98.5 |
| Product Distribution, Wt. Pct. | Feed | | | | |
| $C_1$—$C_4$ N.A. | | 0.7 | 0.5 | 0.6 | 0.8 |
| $C_5$—$C_9$ N.A. | | 0.1 | 0.0 | 0.0 | 0.0 |
| Benzene | | 1.5 | 1.8 | 1.9 | 2.3 |
| Toluene | 1.5 | 2.3 | 2.0 | 2.0 | 2.0 |
| EB | 8.1 | 5.7 | 5.2 | 5.3 | 4.5 |

TABLE 5—continued

| Example No. | 25 | 26 | 27 | 28 |
|---|---|---|---|---|
| P-Xylene | 9.2 | 21.3 | 21.2 | 21.5 | 21.2 |
| M-Xylene | 61.3 | 47.2 | 47.4 | 47.5 | 46.9 |
| O-Xylene | 19.9 | 19.3 | 20.2 | 19.8 | 20.7 |
| $C_9$ + Arom. | | 1.9 | 1.7 | 1.4 | 1.5 |
| TOTAL | 100.0 | 100.0 | 100.0 | 100.0 | 100.0 |

**Claims**

1. Process for isomerizing the xylene content of a charge mixture of aromatic hydrocarbon compounds having eight carbon atoms which mixture contains xylenes and ethylbenzene by contact of the mixture and hydrogen at conversion conditions with a catalyst comprising a hydrogenation component and a crystalline zeolite having a silica to alumina ratio greater than 12 and a constraint index of 1 to 12, characterized by maintaining a conversion temperature of from 287° to 425°C (550° to 800°F):
using a catalyst that has been steam treated to control its acid activity to a measured Alpha value of 45 to 110,
and conducting the contacting at a space velocity effective to produce both a para xylene content of the xylenes at least 98% of that required for thermodynamic equilibrium and a predetermined percentage of conversion of the ethylbenzene content of the charge mixture.

2. The process of Claim 1 wherein the crystalline zeolite is ZSM-5.

3. The process of Claim 1 or 2 wherein the hydrogenation component comprises a Group VIII metal.

4. The process of Claim 3 wherein the hydrogenation metal comprises platinum.

5. The process of any one of Claims 1 to 4 wherein the conversion temperature is maintained at 342° to 398°C (650° to 750°F).

6. The process of any one of Claims 1 to 5 wherein said crystalline zeolite is steamed to an Alpha value of about 60.

7. The process of any one of Claims 1 to 6 wherein the predetermined percentage of ethylbenzene is from 10 to 60.

8. Process for isomerizing the xylene content of a mixture consisting essentially of xylenes and ethylbenzene by contact of the mixture and hydrogen with a steamed catalyst comprising a hydrogenation component and a crystalline zeolite having a silica to alumina ratio greater than 12 and a constraint index of 1 to 12, the contacting being at conversion conditions effective to convert a predetermined percentage of the ethylbenzene in the feed to products of different molecular weight, characterized by
comparing two or more catalysts having different measured Alpha values from 45 to 110 under contact conditions effective to convert the predetermined percentage of ethylbenzene determining therefrom the Alpha value and the temperature in the range of from 287° to 425°C (550° to 800°F) for which the xylene loss is minimized and conducting the process at that temperature with a catalyst having about that determined Alpha value.

9. The process of Claim 8 wherein the predetermined percentage of ethylbenzene is from 10 to 60.

10. The process of Claim 8 or 9 wherein the crystalline zeolite is ZSM-5.

11. The process of any one of Claims 8 or 10 wherein the hydrogenation component comprises a Group VIII metal.

12. The process of Claim 11 wherein the hydrogenation metal comprises platinum.

13. The process of any one of Claims 8 to 12 wherein the determined temperature is in the range of from 342° to 398°C (650° to 750°F).

**Revendications**

1. Procédé pour l'isomérisation du contenu en xylène d'une charge d'un mélange de composés hydrocarbonés aromatiques comprenant 8 atomes de carbone, lequel mélange contient des xylènes et de l'éthylbenzène, par contact du mélange et d'hydrogène, aux conditions de conversion avec un catalyseur comprenant un constituant d'hydrogénation et une zéolite cristalline ayant un rapport

# 0 057 607

silice/alumine supérieur à 12 et un indice de contrainte compris entre 1 et 12, caractérisé:
— en ce que la température de conversion est maintenue comprise entre 257 et 425°C (550 à 800°F);
— en ce que le catalyseur employé a subi un traitement à la vapeur pour que son activité acide soit maintenue à une valeur alpha mesurée de 45 à 110, et
— en ce que le contact est effectué à une vitesse spatiale efficace pour produire à la fois une proportion de para-xylène dans les xylènes au moins égale à 98% de celle qui est requise pour l'équilibre thermodynamique et un pourcentage prédéterminé de conversion de l'éthylbenzène contenu dans le mélange de charge.

2. Le procédé selon la revendication 1, dans lequel la zéolite cristalline est la ZSM-5.

3. Le procédé selon la revendication 1 ou 2, dans lequel le constituant d'hydrogénation comprend un métal du groupe VIII.

4. Le procédé selon la revendication 3, dans lequel le métal d'hydrogénation comprend du platine.

5. Le procédé selon une quelconque des revendications 1 à 4, dans lequel la temperature de conversion est maintenue entre 342 et 398°C (650 et 750°F).

6. Le procédé selon une quelconque des revendications 1 à 5, dans lequel ladite zéolite cristalline est soumise à un traitement à la vapeur jusqu'à une valeur alpha d'environ 60.

7. Le procédé selon une quelconque des revendications 1 à 6, dans lequel le pourcentage prédéterminé d'éthylbenzène est compris entre 10 et 60.

8. Procédé pour l'isomérisation de la teneur en xylène d'un mélange essentiellement formé de xylènes et d'éthylbenzène par contact du mélange et d'hydrogène avec un catalyseur ayant subi un traitement à la vapeur, comprenant un constituant d'hydrogénation et une zéolite cristalline ayant un rapport molaire silice/alumine supérieur à 12 et un indice de contrainte compris entre 1 et 12, le contact se produisant à des conditions de conversion efficaces pour la conversion d'un pourcentage prédéterminé d'éthylbenzène de la charge en des produits de poids moléculaire différent, caractérisé en ce que l'on compare deux ou plusieurs catalyseurs ayant différentes valeurs alpha mesurées comprises entre 45 et 110 dans des conditions de contact efficaces pour convertir le pourcentage prédéterminé de l'éthylbenzène et déterminer, à partir de cela, la valeur alpha et la température comprise entre 287 et 425°C (550° et 800°F) pour laquelle la perte en xylène est rendue minime et effectuer le procédé à cette température à l'aide d'un catalyseur ayant approximativement la valeur alpha déterminée.

9. Le procédé selon la revendication 8, dans laquelle le pourcentage prédéterminé d'éthylbenzène est compris entre 10 et 60.

10. Le procédé selon la revendication 8 ou 9, dans laquelle la zéolite cristalline est la ZSM-5.

11. Le procédé selon une quelconque des revendications 8 ou 10, dans laquelle le constituant d'hydrogénation comprend un métal du groupe VIII.

12. Le procédé selon la revendication 11, dans laquelle le métal d'hydrogénation comprend du platine.

13. Le procédé selon une quelconque des revendications 8 à 12, dans laquelle la température déterminée est comprise entre 342 et 390°C (650 à 750°F).

## Patentansprüche

1. Verfahren zur Isomerisierung des Xylol-Anteils einer Einsatzprodukt-Mischung von aromatischen Kohlenwasserstoffverbindungen, die 8 Kohlenstoffatome aufweisen, wobei diese Mischung Xylole und Ethylbenzol ethält, durch Kontakt der Mischung und Wasserstoff bei Umwandlungsbedingungen mit einem Katalysator, der einen Hydrierbestandteil sowie einen kirstallinen Zeolithen umfaßt, der ein Siliciumdioxid/Aluminiumoxid-Verhältnis von mehr als 12 und einen Grenzwertindex (constraint index) von 1 bis 12 aufweist, gekennzeichnet durch das Aufrechterhalten einer Umwandlungstemperatur von von 287° bis 425°C (550° bis 800°F), Verwendung eines Katalysators, der dampfbehandelt wurde, um seine Säureaktivität auf einen gemessenen $\alpha$-Wert von 45 bis 110 einzustellen, und Durchführen des Kontaktierens bei einer Raumgeschwindigkeit, die ausreicht, sowohl einen para-Xylol-Gehalt der Xylole von wenigstens 98% des für das thermodynamische Gleichgewicht erforderlichen herzustellen sowie eine vorgegebene prozentuale Umwandlung des Ethylbenzol-Anteils der Einsatzprodukt-Mischung zu erzielen.

2. Verfahren nach Anspruch 1, bei dem der kristalline Zeolith ZSM-5 ist.

3. Verfahren nach Anspruch 1 oder 2, bei dem der Hydrierbestandteil ein Metall der Gruppe VIII umfaßt.

4. Verfahren nach Anspruch 3, bei dem das Hydriermetall Platin umfaßt.

5. Verfahren nach einem beliebigen der Ansprüche 1 bis 4, bei dem die Umwandlungstemperatur auf 342° bis 398°C (650° bis 750°F) gehalten wird.

6. Verfahren nach einem beliebigen der Ansprüche 1 bis 5, bei dem der genannte kristalline Zeolith auf einen $\alpha$-Wert von etwa 60 gedämpft wurde.

7. Verfahren nach einem beliebigen der Ansprüche 1 bis 6, bei dem der vorgegebene prozentuale Anteil des Ethylbenzols von 10 bis 60 beträgt.

8. Verfahren zur Isomerisierung des Xylol-Anteils einer Mischung, die im wesentlichen aus

17

**0 057 607**

Xylolen und Ethylbenzol besteht, durch Kontakt der Mischung und von Wasserstoff mit einem gedämpften Katalysator, der einen Hydrierbestandteil und einen kristallinen Zeolithen umfaßt, der ein Siliciumdioxid/Aluminiumoxid-Verhältnis von mehr als 12 und einen Grenzwertindex (constraint index) von 1 bis 12 aufweist, wobei das Kontaktieren bei Umwandlungsbedingungen erfolgt, die im Hinblick auf die Umwandlung eines vorgegebenen prozentualen Anteils des Ethylbenzols in dem Einsatzprodukt in Produkte unterschiedlichen Molekulargewichts wirksam sind, gekennzeichnet durch

Vergleichen von zwei oder mehr Katalysatoren mit unterschiedlichen gemessenen $\alpha$-Werten von 45 bis 110 unter Kontaktbedingungen, die im Hinblick auf die Umwandlung des vorgegebenen prozentualen Anteils des Ethylbenzols wirksam sind,

Ermitteln daraus den $\alpha$-Wert und die Temperatur im Bereich von 287° bis 425°C (550° bis 800°F), für die der Xylol-Verlust minimal wird und

Durchführen des Verfahrens bei dieser Temperatur mit einem Katalysator, der etwa den ermittelten $\alpha$-Wert aufweist.

9. Verfahren nach Anspruch 8, bei dem der vorgegebene prozentuale Anteil des Ethylbenzols von 10 bis 60 beträgt.

10. Verfahren nach Anspruch 8 oder 9, bei dem der kristalline Zeolith ZSM-5 ist.

11. Verfahren nach einem beliebigen der Ansprüche 8 oder 10, bei dem der Hydrierbestandteil ein Metall der Gruppe VIII umfaßt.

12. Verfahren nach Anspruch 11, bei dem das Hydriermetall Platin umfaßt.

13. Verfahren nach einem beliebigen der Ansprüche 8 bis 12, bei dem die ermittelte Temperatur im Bereich von 342° bis 398°C (650° bis 750°F) liegt.

18

FIG. 1

0 057 607

XYLENE LOSS VS. TEMPERATURE

FIG. 2